# EUROPÄISCHE PATENTSCHRIFT

(11) **EP 3 055 284 B1**
(45) Veröffentlichungstag und Bekanntmachung des Hinweises auf die Patenterteilung: **30.08.2017**
(21) Anmeldenummer: 14777662.9
(22) Anmeldetag: 02.10.2014
(51) Int. Cl.: C07C 209/36

(54) **VERFAHREN ZUR HYDRIERUNG VON DINITROTOLUOL IN DER GASPHASE ZUR HERSTELLUNG VON TOLUYLENDIAMIN**
PROCESS FOR THE GAS PHASE HYDROGENATION OF DINITROTOLUENE FOR THE PREPARATION OF DIAMINOTOLUENE
PROCÉDÉ POUR L'HYDROGÉNATION EN PHASE GAZEUSE DU DINITROTOLUÈNE POUR LA PRÉPARATION DE TOLUYLÈNEDIAMINE

(30) Priorität: 08.10.2013 EP 13187648
(43) Veröffentlichungstag der Anmeldung: 17.08.2016
(73) Patentinhaber: Covestro Deutschland AG, 51373 Leverkusen (DE)
(72) Erfinder: PENNEMANN, Bernd, 51467 Bergisch Gladbach (DE); MOUSSA, Amgad Salah, 50933 Köln (DE); MAHR, Bastian, 51467 Bergisch Gladbach (DE)
(74) Vertreter: Levpat
(86) Internationale Anmeldenummer: PCT/EP2014/071125
(87) Internationale Veröffentlichungsnummer: WO 2015/052068

(56) Entgegenhaltungen:
- DE-A1- 3 734 344
- GB-A- 832 939

## Beschreibung

Die Erfindung betrifft ein großtechnisch umsetzbares Verfahren zur Gasphasenhydrierung von Dinitrotoluol (DNT), bei dem ein DNT-haltiger Strom, ggf. in Gegenwart eines Zerstäubungsgases, in einen Wasserstoff-haltigen Trägergasstrom verdüst wird, der erhaltene, im Wesentlichen gasförmige, Strom von unverdampften Flüssigkeitstropfen befreit und der resultierende Gasstrom katalytisch zu Toluylendiamin hydriert wird.

Aromatische Amine sind wichtige Zwischenprodukte, welche preiswert und in großen Mengen zur Verfügung stehen müssen. Daher müssen z. B. für die Hydrierung von Dinitrotoluol (nachfolgend auch DNT) Anlagen mit sehr großen Kapazitäten gebaut werden. Das Hydrierprodukt Toluylendiamin (nachfolgend auch TDA) ist ein wichtiges Intermediat in der Herstellung von Toluylendiisocyanat, welches in der Polyurethanchemie große Bedeutung hat. Es gibt zahlreiche Veröffentlichungen zur Herstellung von Toluylendiamin. Der weitaus größte Teil des Standes der Technik befasst sich mit der Hydrierung von Dinitrotoluol in der Flüssigphase. Bekannt sind zum einen "einphasige" Verfahren, entweder ohne weiteres Lösungsmittel (siehe bspw. US 3,093,685) oder unter Verwendung von Lösungsmitteln, die sowohl DNT als auch das gebildete TDA-Wasser-Gemisch lösen, wie bspw. einfachen aliphatischen Alkoholen (z. B. Methanol). Daneben gibt es zum anderen "zweiphasige" Verfahren, bei denen Lösungsmittel (z. B. Kohlenwasserstoffe) eingesetzt werden, die DNT, nicht jedoch das gebildete TDA-Wasser-Gemisch lösen, sodass es zu einer Phasenseparation kommt; siehe z. B. GB 1 490 313**.** Der Katalysator (bspw. Pd/C, Raney Ni, Ni/SiO₂ u. a.) wird für gewöhnlich in der flüssigen Phase aufgeschlämmt (daher auch "Slurry-Phasenverfahren" genannt). Als Reaktoren kommen bspw. Schlaufenreaktoren oder gerührte Kessel in Frage (siehe z. B. US 2011/295039 A1). Alle derzeit großtechnisch relevanten Verfahren arbeiten in der Flüssigphase. Flüssigphasenhydrierverfahren bei höheren Temperaturen und die Gasphasenhydrierung von Dinitrotoluol spielen aufgrund der potenziellen Gefahren infolge der thermischen Instabilität insbesondere des technischen Dinitrotoluols großtechnisch keine Rolle. Die Gasphasen-Hydrierung von wenig flüchtigen und/oder temperaturempfindlichen Nitroaromaten wird in der Literatur kritisch gesehen (siehe z. B. Cartolano, A. R. and Vedage, G. A., 2004, Amines by Reduction, Kirk-Othmer, Encyclopedia of Chemical Technology, John Wiley & Sons Inc; 5. Auflage (31. Januar 2004), Vol. 2, Seite 478 und Seite 484, Online-ISBN: 9780471238966).

GB 599,252 und US 3,13,6818 beschreiben Verfahren zur Herstellung von aromatischen Monoaminen, insbesondere Anilin, in der Gasphase durch Hydrierung in einem Wirbelschichtreaktor. Da Mononitro-Aromaten wesentlich stabiler als Dinitro-Aromaten sind, stellt die unkontrollierte thermische Zersetzung kein wesentliches Problem dar. Vor der Gasphasenhydrierung von Ausgangsstoffen mit höheren Anteilen von Dinitro-Verbindungen wird in GB 599,252 ausdrücklich gewarnt.

DE-AS 1 809 711 befasst sich mit einem Verfahren zur Gasphasenhydrierung von Nitroverbindungen und widmet sich insbesondere dem Problem des gleichmäßigen Einbringens von flüssigen Nitroverbindungen in einen heißen Gasstrom durch Verdüsen, bevorzugt an verengten Stellen unmittelbar vor dem Reaktor. Die Gefahr einer möglicherweise unvollständigen Verdampfung der Nitroverbindung wird nicht erwähnt. Die Schrift spricht zwar in allgemeiner Weise von Nitroverbindungen, gibt aber nur für Nitrobenzol ein konkretes Beispiel. Die in der Schrift genannten Verfahrensparameter sind für Nitrobenzol optimiert.

In DE-OS 3 636 984 wird ein Verfahren zur gekoppelten Produktion von Nitro- und Dinitroaromaten aus den entsprechenden Kohlenwasserstoffen durch Nitrierung und deren nachfolgende Hydrierung beschrieben. Die Hydrierung erfolgt in der Gasphase bei Temperaturen von 176 bis 343,5 °C. Es wird eine Apparatur zur Gasphasenhydrierung beschrieben, die im Wesentlichen aus zwei hintereinander geschalteten Reaktoren mit Zwischenkühlung und Eduktzwischeneinspeisung besteht, auf deren Größe und Aufbau nicht eingegangen wird. Die Problematik der Zersetzung von Dinitrotoluol wird in der Schrift nicht behandelt.

Die Schriften EP 0 696 573 A1**,** EP 0 696 574 A1**,** EP 0 748 789 A1**,** EP 0 748 790 A1 **sowie** DE 10 2006 035 203 A1 befassen sich mit einem unter rein adiabaten Bedingungen durchgeführten Gasphasen-Verfahren zur Hydrierung aromatischer Nitroverbindungen. EP 0696574 A1 beschreibt den Prozess zur Herstellung von aromatischen Aminen, in welchem der Katalysator unter adiabaten Bedingungen mit einem Gasgemisch bestehend aus Nitroaromaten und Wasserstoff angeströmt wird, in ganz allgemeiner Weise. In den Verfahren gemäß der übrigen genannten Schriften werden durch Änderung verschiedener Parameter jeweils bestimmte Vorteile erzielt. Die Verfahren sind anwendbar für Nitroaromaten der allgemeinen Formel 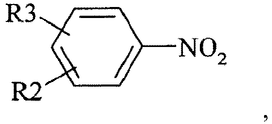 wobei R2 und R3 unter anderem auch für eine Methylgruppe stehen können. Der Schwerpunkt besagter Schriften liegt jedoch auf Anilin (die Beispiele befassen sich mit der Hydrierung von Nitrobenzol). Auf Besonderheiten der Dinitrotoluolhydrierung gehen die genannten Schriften nicht ein.

Speziell mit der Hydrierung von Dinitroverbindungen in der Gasphase befasst sich GB 832,939**.** Diese Schrift offenbart die Verwendung von Nickelsulfid-Katalysatoren auf einem Aluminiumoxid-Trägermaterial. Deren Einsatz ermöglicht gemäß den Angaben der Schrift eine unerwartet schnelle Reaktion in hervorragenden Ausbeuten. Auf verfahrenstechnische Details zur Verdampfung geht die Schrift nicht ein. Die Hydrierung wird bei Umgebungsdruck und Temperaturen um 220 °C (vgl. Beispiele), d. h. unterhalb der Zersetzungstemperatur von flüssigem reinem Dinitrotoluol, betrieben.

DE 3734344 A1 beschreibt die Umsetzung von Dinitrotoluol (DNT) in der Gasphase zu Toluylendiamin (TDA). DNT wird in einem inerten, heißen Trägergas innerhalb von 2 bis 120 Sekunden zu einem 150 bis 250 °C heißen Gemisch aus verdampftem DNT und Trägergas verdampft. Als geeignete Verdampfertypen werden Dünnschichtverdampfer mit glatt konstruierten Rohren, Kurzwegverdampfer, Fallfilmverdampfer ohne Flüssigkeitskreislauf und einsträngige Wendelrohrverdampfer genannt. Die geringe Flüchtigkeit und hohe Zersetzlichkeit von DNT sowie das damit verbundene Explosionsrisiko werden erwähnt. Maßnahmen zur Zersetzungsvermeidung oder zur Vermeidung der Akkumulation von schwerersiedenden, thermisch empfindlichen Verunreinigungen werden nicht beschrieben, da sie vermutlich aufgrund der kurzen Versuchsdauer von einigen Stunden und möglicherweise aufgrund der Reinheit der eingesetzten Ausgangsstoffe bei den durchgeführten Experimenten nicht problematisch waren. Das mögliche Vorhandensein nicht verdampfbarer Anteile im DNT wird nur insofern erwähnt, als das Verdampfungsverfahren theoretisch zur Trennung in DNT und nicht verdampfbarer Anteile nutzbar sein kann. Die Hydrierung wird gemäß dieser Schrift im Temperaturbereich von 200 bis 450 °C und bevorzugt bei Normaldruck durchgeführt.

Aus den literaturbekannten Quellen, die ausdrücklich auf die Möglichkeit der Gasphasenhydrierung von DNT hinweisen, lässt sich kein technisches Konzept für eine wirtschaftliche Umsetzung in großem Maßstab ableiten. Die Literatur (insbesondere GB 832939 und DE 3734344 A1) geht weder auf die bei der Realisierung eines großtechnischen DNT-Hydrierverfahrens zu überwindenden Hürden noch auf die Nutzbarmachung der mit einem solchen Verfahren potenziell verbundenen Vorteile ein.

Für die großtechnische Produktion von TDA nach einem Gasphasenverfahren ist zu beachten, dass DNT technischer Reinheit einen höheren Anteil an schwer flüchtigen Begleitkomponenten aufweisen kann als DNT, das in kleinen Chargen für Laborversuche eingesetzt wird. Aus wirtschaftlichen Gründen ist es anzustreben, technisch reines DNT ohne aufwändige und teure Vorreinigung einsetzen zu können. Dies erfordert besondere Maßnahmen bei der Verdampfung von DNT in technischen Produktionsanlagen.

Keine der oben genannten Druckschriften gibt einen Hinweis darauf, dass systematische Schritte unternommen wurden, um eine sichere Verdampfung und Gasphasenhydrierung von Dinitrotoluol *technischer Reinheit für lange Betriebslaufzeiten und im technischen Maßstab* zu ermöglichen, ohne dass DNT oder seine Begleitkomponenten sich unkontrolliert zersetzen. Ebenso wenig werden Angaben zu
a) kritischen Temperaturgrenzen zur Vermeidung der thermischen Zersetzung des DNT,
b) dem Umgang mit schwer oder nicht verdampfbaren Begleitstoffen mit Gefahrenpotenzial (z. B. Pikrinsäure, Kresole, Trinitrotoluol (TNT)),
c) der Vermeidung der Akkumulation von unverdampften Stoffen, die im unverdampften Zustand ein Gefahrenpotenzial haben (DNT, TNT, etc.) und
d) der Sicherstellung des vollständigen Umsatzes von DNT, bevor der Produkt-Gasstrom abgekühlt wird.
gemacht.

Bei der Hydrierung von DNT werden große Mengen Energie frei. Im herkömmlichen Flüssigphasenverfahren wird bei einem absoluten Druck von ca. 20 bis 100 bar Wasserstoff zudosiert, und die Reaktoren werden bei diesem Druck betrieben; siehe z. B. US 2008/0146847 A1 (100 bar Druck und eine Temperatur von 150 °C). Aufgrund des niedrigen Temperaturniveaus ist die Nutzung der abzuführenden Energie nur sehr eingeschränkt möglich bzw. wirtschaftlich. Könnte die Hydrierreaktion bei höherer Temperatur durchgeführt werden, so dass höhergespannter Dampf erzeugbar wäre, hätte das großen ökonomischen Nutzen. Dies gilt insbesondere für Verbundsysteme aus mehreren Produktionsanlagen, in denen Dampf, der in einem Prozess anfällt, in anderen Prozessen (bspw. zur Aufheizung der Edukte auf die Reaktionstemperatur) genutzt werden kann. Kürzlich wurde darüber berichtet, die Flüssigphasenhydrierung ohne Lösungsmittel bei einer Temperatur durchzuführen, die die Produktion von Dampf auf einem Druckniveau von 4 bar ermöglicht (US 2011/0275858 A1). Hierzu wurde die Hydrierung bei einer Temperatur von 185 °C durchgeführt, was unter folgenden Bedingungen auf sichere Art und Weise möglich war:
1. Es wurde ein Reaktor mit einer internen Wärmeübertragungsoberfläche und einem externen Kreislaufsystem mit Wärmeabfuhr eingesetzt.
2. DNT wurde mit einer Treibdüse unterhalb der Flüssigkeitsoberfläche in die Katalysatorsuspension eingeführt.
3. Die durchschnittliche DNT-Konzentration im Reaktor wurde auf einen Wert von kleiner 1000 ppm begrenzt.
4. Die Wasserstoffkonzentration inkl. des Wasserstoffs im externen Kreislauf wurde auf einen Wert von größer 1 Vol.-%, bevorzugt größer 3 Vol.-%, eingestellt.

Die Einhaltung dieser Bedingungen ist für das beschriebene Verfahren essentiell, u. A. weil sich Nitro- und Nitrosoverbindungen in Gegenwart von TDA bei erhöhten Temperaturen explosionsartig zersetzen können (DE 10 2005 008 613 A1). Insbesondere die dritte und vierte Bedingung können jedoch bei einer großtechnischen Produktion bei einer Temperatur von 185 °C oder größer zu großen praktischen Problemen bei der Auslegung und dem Betrieb eines Flüssigphasenprozesses führen. Die Abstimmung der einzelnen Parameter aufeinander ist schwierig zu realisieren. Wie nachfolgend noch näher erläutert wird, ermöglicht die vorliegende Erfindung die Einhaltung einer Temperatur von 185 °C oder größer, sodass hochgespannter Dampf erhalten werden kann, in einem Gasphasenprozess ohne vergleichbare praktische Limitierungen.

Daneben haben Gasphasenverfahren noch eine Reihe von anderen Vorteilen. So ist die Abtrennung des Katalysators vom Produkt einfacher, da das Produkt den Reaktor gasförmig verlässt während der Katalysator im Reaktor verbleibt. Auch ist die Hochskalierung des Prozesses im Fall von Gasphasenreaktoren einfacher als bei den im Slurry-Phasenverfahren üblichen Reaktoren. Da im Gasphasenprozess nicht mechanisch gerührt werden muss, ist die Gefahr von Anlagenausfällen durch Anbackungen am Rührer sowie der Energieverbrauch geringer. Auch lassen sich Gasphasenreaktoren einfacher reinigen als Rührkessel.

Ein weiterer Vorteil einer großtechnischen TDA-Synthese in der Gasphase ergibt sich aus der Möglichkeit, das gasförmig anfallende Produkt durch eine fraktionierende Kondensation bei unterschiedlichen Temperaturen auf einfache Weise einer Vortrennung (inkl. Isomerentrennung) zu unterziehen. Jede der so erhaltenen Kondensatfraktionen kann der nachfolgenden Destillationssequenz an einer anderen, genau auf diese Fraktion abgestimmten Stelle zugeführt werden. Hierdurch ergibt sich die Möglichkeit, die Destillation im Vergleich mit der üblichen Destillation des Produkts eines Flüssigphasenverfahrens (bis zu sechs Kolonnen, siehe bspw. US 6,359,177 B1) erheblich zu vereinfachen.

Es bestand daher ein Bedarf an der Bereitstellung eines Verfahrens für die Durchführung der Hydrierung von DNT technischer Reinheit in der Gasphase, welches sich in großtechnischem Maßstab umsetzen lässt und es so ermöglicht, die vielfältigen Vorteile einer Gasphasenhydrierung, insbesondere die Möglichkeit der Gewinnung hochgespanntens Dampfes und die vereinfachte Aufarbeitung durch fraktionierende Kondensation, tatsächlich nutzbar zu machen und dadurch die Energieeffizienz des Verfahrens deutlich zu steigern. Die besondere Herausforderung dabei war es, die Gefahr der unkontrollierten Zersetzung von DNT und seiner Begleitkomponenten zu vermeiden.

Dem vorstehend Gesagten Rechnung tragend ist ein Gegenstand der vorliegenden Erfindung ein kontinuierliches Verfahren zur Herstellung von Toluylendiamin durch Hydrierung von Dinitrotoluol in der Gasphase umfassend die Schritte
(I) Einsprühen eines Dinitrotoluol umfassenden Stromes (**1, 11, 12**) in einen wasserstoffhaltigen Trägergasstrom (**2, 21, 22**) in einer Verdampfungsapparatur (**1000**, **1010, 1020**) wobei
   a) die Temperatur des Dinitrotoluol umfassenden Stromes (**1, 11, 12**) 70 °C bis 150 °C, bevorzugt 80 °C bis 100 °C, und die Temperatur des wasserstoffhaltigen Trägergasstroms (**2, 21, 22**) 140 °C bis 300 °C, bevorzugt 180 °C bis 240 °C, beträgt,
   b) der absolute Druck des Dinitrotoluol umfassenden Stromes (**1, 11, 12**) 3,0 bar bis 30 bar, bevorzugt 4,0 bar bis 20 bar, und der absolute Druck des wasserstoffhaltigen Trägergasstroms (**2, 21, 22**) 1,0 bar bis 10 bar, bevorzugt 3,0 bar bis 6,0 bar, beträgt, wobei der Druck des Dinitrotoluol umfassenden Stromes (**1, 11, 12**) höher ist als der des wasserstoffhaltigen Trägergasstroms (**2**, **21, 22**), bevorzugt um 0,01 bar bis 10 bar höher,
   c) das molare Verhältnis von Wasserstoff zu DNT > 6,0 : 1 bis 900 : 1, bevorzugt 60 : 1 bis 500 : 1, beträgt,
   sodass Dinitrotoluol innerhalb von 0,010 s bis 100 s, bevorzugt innerhalb von 0,010 s bis 3,0 s, verdampft wird, wobei mindestens 95,0 Massen-%, bevorzugt mindestens 99,5 Massen-%, jeweils bezogen auf die Gesamtmasse allen im Dinitrotoluol umfassenden Strom (**1, 11, 12**) enthaltenen Dinitrotoluols, in die Gasphase überführt werden, und auf diese Weise ein Dinitrotoluol und Wasserstoff umfassender, im Wesentlichen gasförmiger Strom (**3, 31, 32**) erhalten wird, der ggf. noch Anteile an unverdampften, schwerflüchtigen Begleitkomponenten des Dinitrotoluols enthält;
(II) Abtrennung oder gezielte Zersetzung, bevorzugt Abtrennung, der in dem Dinitrotoluol und Wasserstoff umfassenden, im Wesentlichen gasförmigen Strom aus Schritt (I) (**3, 31, 32**) enthaltenen Flüssigkeitsstropfen in einer Vorrichtung (**2000, 2010, 2020**) so, dass der resultierende, an Flüssigkeitstropfen abgereicherte Dinitrotoluol und Wasserstoff umfassende Gasstrom (**4, 41, 42**) bevorzugt maximal 1000 ppm, besonders bevorzugt maximal 500 ppm, ganz besonders bevorzugt maximal 100 ppm, außerordentlich ganz besonders bevorzugt maximal 50 ppm an unverdampften Tropfen enthält, bezogen auf die Gesamtmasse allen im Dinitrotoluol umfassenden Strom (**1, 11, 12**) enthaltenen Dinitrotoluols;
(III) Umsetzung des in dem an Flüssigkeitstropfen abgereicherten Dinitrotoluol und Wasserstoff umfassenden Gasstroms (**4, 41, 42**) enthaltenen Dinitrotoluols mit Wasserstoff in wenigstens einem Reaktionsraum (**3000, 3010, 3020**) in Gegenwart eines Katalysators (**100, 110, 120**) bei einem absoluten Druck von 1,0 bar bis 10 bar, bevorzugt von 3,0 bar bis 6,0 bar, einer Temperatur von 140 °C bis 300 °C, bevorzugt von 180 °C bis 270 °C, und einer Verweilzeit im Reaktionsraum von 0,1 s bis 10 s, bevorzugt von 1,0 s bis 5,0 s, sodass ein Toluylendiamin enthaltender Gasstrom (**5, 51, 52**) erhalten wird,
(IV) Trennung des in Schritt (III) nach Durchlaufen des letzten Reaktionsraums (**3000, 3020**) erhaltenen Toluylendiamin enthaltenden Gasstroms (**5, 52**) in eine Toluylendiamin umfassende Flüssigphase (**6, 6a, 6b, 6c**) und eine Wasserstoff umfassende Gasphase (7) durch Kondensation, bevorzugt in einer Kondensationsapparatur (**4000**), besonders bevorzugt durch mehrstufige Kondensation (**4010, 4020, 4030**) mit von Stufe zu Stufe sinkender Temperatur;
(V) Rückführung wenigstens eines Teils der in Schritt (IV) erhaltenen Wasserstoff umfassenden Gasphase (**7**) in die erste Verdampfungsapparatur (**1000, 1010**) von Schritt (I).

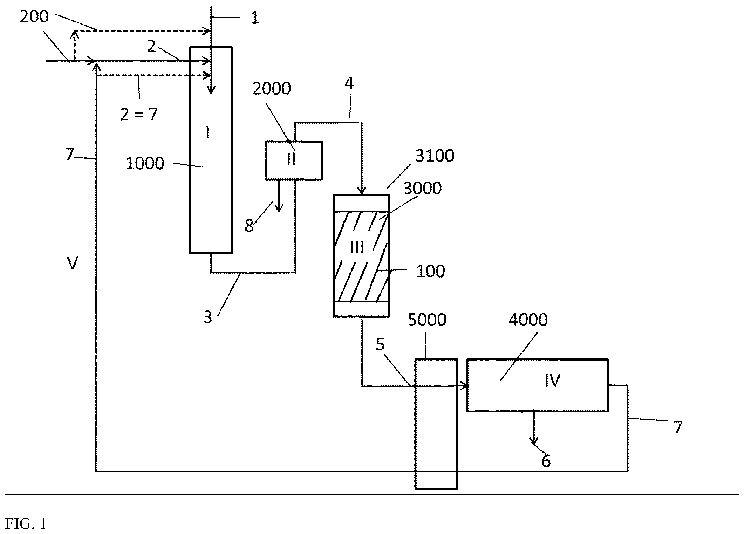

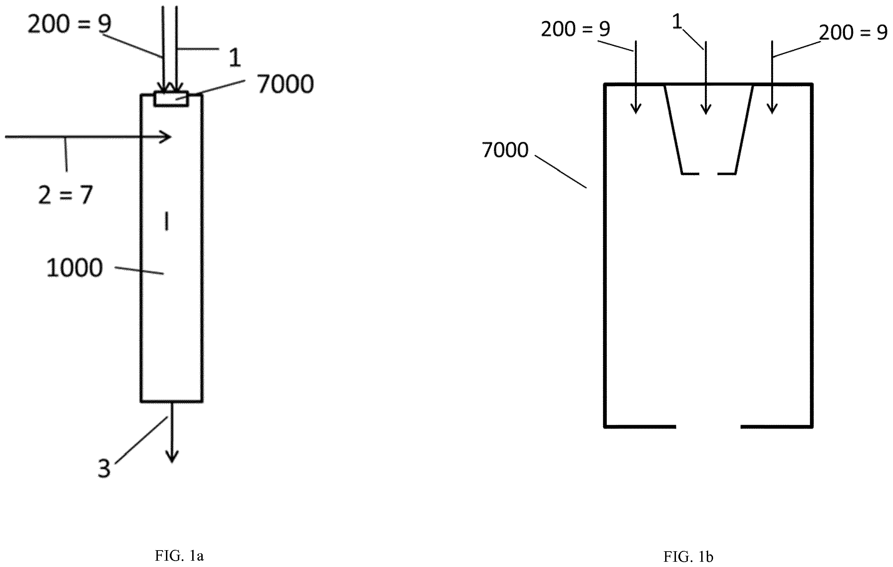

Unter einer *Verdampfungsapparatur* wird in diesem Zusammenhang jede Vorrichtung verstanden, die sich zum Einsprühen des DNT-Stroms **1** (**11, 12**) in den Trägergasstrom (den "Einsatzwasserstoff"-Strom) **2** (**21, 22**) und zum möglichst vollständigen Verdampfen des DNTs eignet. In der Ausführungsform gemäß FIG.1 - durchgezogene Linien - ist der "Einsatzwasserstoff"-Strom **2** beispielsweise eine Mischung aus "Kreisgaswasserstoff" **7** und Frischwasserstoff **200**; in der Ausführungsform gemäß FIG. 1 mit gestrichelten Linien ist er identisch mit dem Kreisgaswasserstoff (**7**). Die Verdampfungsapparatur umfasst mindestens eine Vorrichtung zum Versprühen des Stroms **1** (**11, 12**) in den Strom **2** (**21, 22**). Im einfachsten Fall umfasst die Verdampfungsapparatur lediglich eine Rohrleitung, durch die Strom **2** (**21, 22**) fließt, und in welche eine Vorrichtung, durch die Strom **1** (**11, 12**) eingesprüht wird, mündet. Eine "Vorrichtung zum Versprühen" ist bevorzugt eine Düse (siehe weiter unten für Details).

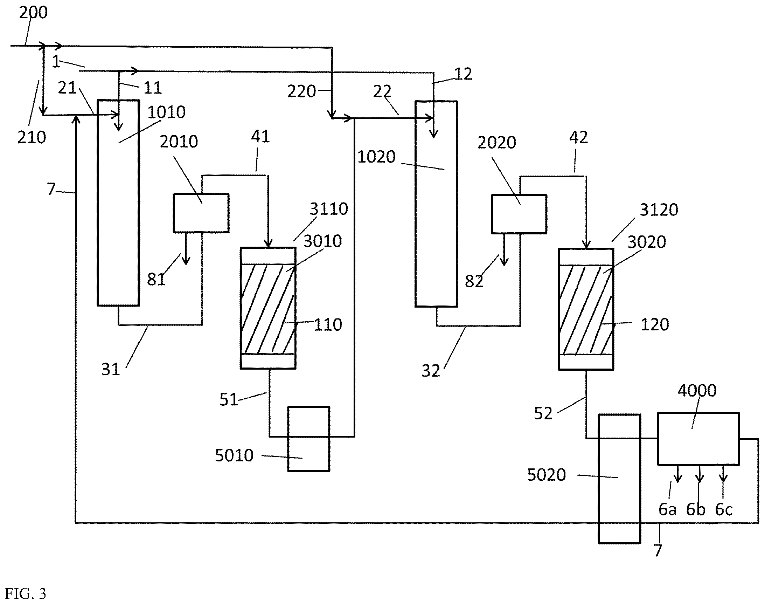

Die *erste Verdamfpungsapparatur (**1000, 1010**)* bezeichnet bei Einsatz von mehreren in Serie geschalteten Reaktionsräumen und damit auch bei Einsatz von mehreren in Strömungsrichtung des Reaktionsgemisches hintereinander angeordneten Verdampfungsapparaturen (vgl. z. B. FIG. 3) die in Strömungsrichtung des Reaktionsgemisches erste Verdampfungsapparatur, also in der Ausführungsform nach FIG. 3 die Verdampfungsapparatur **1010.** Bei mehreren parallel geschalteten Reaktionsräumen und somit auch mehreren parallel geschalteten Verdampfungsapparaturen sind alle in Strömungsrichtung des Reaktionsgemisches angeordneten ersten Verdampfungsapparaturen "erste Verdampfungsapparaturen" im Sinne von Schritt (V). Ist nur ein Reaktionsraum und somit auch nur eine Verdampfungsapparatur vorhanden (vgl. z. B. FIG. 1), so ist diese Verdampfungsapparatur natürlich auch "die erste" Verdampfungsapparatur im Sinne von Schritt (V).

Dabei beziehen sich die Angaben, dass *mindestens 95,0 Massen-%, bezogen auf die Gesamtmasse allen im Dinitrotoluol umfassenden Strom (**1, 11, 12**) enthaltenen Dinitrotoluols, in die Gasphase überführt werden* [Schritt (I)] und *der Gasstrom **4** (**41, 42**) bevorzugt maximal 1000 ppm an unverdampften Tropfen, bezogen auf die Gesamtmasse allen im Dinitrotoluol umfassenden Strom (****1*,** ***11, 12**) enthaltenen Dinitrotoluols, enthält* [Schritt (II)] jeweils auf DNT als solches, d. h. ohne Berücksichtigung der in DNT technischer Reinheit immer enthaltenen Verunreinigungen. Wenn Strom **1** (**11, 12**) beispielsweise ein Dinitrotoluol 98%iger Reinheit ist, welches mit *a* kg/h der Verdampfung in Schritt (I) zugeführt wird, so werden mit Strom **3** (**31, 32**) mindestens 0,95 · 0,98 · *a* kg/h gasförmigen Dinitrotoluols der Tropfenentfernung in Schritt (II) zugeführt. Der Resttropfengehalt im an Flüssigkeitstropfen abgereicherten Dinitrotoluol und Wasserstoff umfassenden Gasstrom (**4, 41, 42**) wird bevorzugt durch laseroptische Messung, mittels kapazitiver Messtechnik oder durch Entnahme eines repräsentativen Teilstroms und Bestimmung seines Tropfengehalts mittels vorgenannter Techniken oder mittels Auffangen und Wägung der Tropfen bestimmt. Die zuvor beschriebenen, dem Fachmann geläufigen Methoden liefern im Allgemeinen im Rahmen üblicher, der Zuverlässigkeit des Ergebnisses nicht abträglicher Fehlertoleranzen die gleichen Resultate. Für den Fall, dass verschiedene Messmethoden dennoch signifikant unterschiedliche Werte liefern sollten, ist erfindungsgemäß das Ergebnis der laseroptischen Messung maßgeblich.

Unter *Reaktionsraum* (Schritt (III)) wird im Rahmen der vorliegenden Erfindung der Raum verstanden, in welchem DNT und Wasserstoff in Gegenwart des Katalysators miteinander reagieren. Der Reaktionsraum befindet sich in einer technischen Vorrichtung zur Durchführung von chemischen Reaktionen, dem *Reaktor.* Im Fall eines vollständig mit Katalysator (beispielsweise in Form einer Schüttung aus Katalysatorkugeln) ausgefüllten Reaktors ist der Reaktionsraum identisch mit dem Innenvolumen des Reaktors. Wenn mehrere Reaktionsräume vorhanden sind, können diese seriell oder parallel geschaltet sein. Der *letzte Reaktionsraum* bezeichnet bei mehreren in Serie geschalteten Reaktionsräumen (vgl. z. B. FIG. 3) den in Strömungsrichtung des Reaktionsgemisches letzten Reaktionsraum, also in der Ausführungsform nach FIG. 3 den Reaktionsraum **3020.** Bevorzugt wird nur der in diesem Reaktionsraum erhaltene Toluylendiamin enthaltende Gasstrom in Gas- (7) und Flüssigphase (6) getrennt. Bei mehreren parallel geschalteten Reaktionsräumen sind alle in Strömungsrichtung des Reaktionsgemisches angeordneten letzten Reaktionsräume "letzte Reaktionsräume" im Sinne von Schritt (IV). In diesem Fall ist es bevorzugt, die mehreren so erhaltenen Toluylendiamin enthaltenden Gasströme vor der Kondensation zu vereinigen. Ist nur ein Reaktionsraum vorhanden (vgl. z. B. FIG. 1) ist dieser natürlich auch "der letzte" Reaktionsraum im Sinne von Schritt (IV).

*Verweilzeit im Reaktionsraum* meint im Rahmen der vorliegenden Erfindung den Quotienten aus dem für den Gasstrom durchströmbaren Volumen des Reaktionsraums und dem pro Zeiteinheit aus dem Reaktionsraum austretenden Volumenstrom.

Nachstehend wird die Erfindung im Detail beschrieben. Verschiedene Ausführungsformen sind, sofern sich aus dem Kontext nicht eindeutig das Gegenteil ergibt, beliebig miteinander kombinierbar.

Es wurde überraschenderweise gefunden, dass gasförmige Mischungen aus verdampftem DNT und einem wasserstoffhaltigen Trägergas bei gleicher (hoher) Temperatur thermisch stabiler sind als flüssiges DNT oder Lösungen von DNT hoher Konzentration. Dies gilt selbst in Gegenwart von TDA, mit dem DNT prinzipiell unerwünschte Reaktionen eingehen kann. Wesentlicher Bestandteil des Verfahrens ist daher die ausreichend vollständige Verdampfung von DNT. Das erfindungsgemäße Verfahren ermöglicht es, DNT zu mindestens 99,90 %, bevorzugt zu mindestens 99,95 %, besonders bevorzugt zu mindestens 99,99 %, ganz besonders bevorzugt zu mindestens 99,995 %, in die Gasphase zu überführen (Schritte (I) und (II)), ehe es im Reaktionsraum in Gegenwart des Katalysators zu TDA hydriert wird (Schritt (III)). Die maximal mögliche Menge an unverdampften Tröpfchen von 0,10 %, bezogen auf die Masse allen in **1** vorhandenen Dinitrotoluols, ist so gering, dass keine Zersetzungsreaktionen in gefährlichem Ausmaß zu befürchten sind. Überall dort, wo gasförmiges DNT vorhanden ist, d. h. in der Verdampfungsapparatur sowie im Reaktionsraum, darf eine Temperatur von 300 °C nicht überschritten werden; zudem muss die Verweilzeit gering gehalten werden.

Die Ströme **1** (DNT) und **200** (Frischwasserstoff) können neben den wesentlichen Bestandteilen DNT bzw. Wasserstoff noch weitere enthalten. Insbesondere kann es vorteilhaft sein, das DNT technischer Reinheit in einem geeigneten unter den Reaktionsbedingungen inerten Lösungsmittel zu lösen, welches mit verdampft wird. Geeignete Lösungsmittel sind Alkohole (bevorzugt ausgewählt aus Methanol, Ethanol und Isopropanol). Wenn ein Lösungsmittel eingesetzt wird, so beträgt der Anteil an technischem DNT in der Lösung (**1**) bevorzugt > 0 Massen-% bis ≤ 50 Massen-%, bezogen auf die Gesamtmasse von **1.** Auch der Wasserstoff muss nicht in Substanz eingesetzt werden, sondern kann mit anderen Gasen, die unter den Reaktionsbedingungen der Hydrierung inert sind, verdünnt werden. Dies kann entweder so geschehen, dass der Wasserstoff vor Vermischung mit dem DNT-haltigen Strom **1** mit einem Inertgas vermischt wird, oder Wasserstoff und Inertgas werden getrennt zugegeben. Als inerte Gase eignen sich beispielsweise Edelgase, Wasserdampf, CO₂, Stickstoff; bevorzugt sind Stickstoff oder Wasserdampf. Wird der Wasserstoff vor Kontakt mit dem DNT-haltigen Strom **1** mit solchen Gasen verdünnt, so beträgt sein Anteil im Strom **2** bevorzugt minimal 3 Mol-%, bezogen auf die Gesamtstoffmenge aller in Strom **2** enthaltenen Verbindungen. Zum Beispiel kann auch Synthesegas direkt verwendet werden, ohne den enthaltenen Wasserstoff auf hohe Reinheiten aufzureinigen. Wird ein kondensierbares Verdünnungsgas (z. B. Wasserdampf) verwendet, hat dies den Vorteil, dass der Verdichtervolumenstrom im Vergleich zu nicht kondensierbarem Trägergas sinkt.

Das vorliegende Verfahren ermöglicht **in Schritt (I)** die Verdampfung von DNT technischer Reinheit im großtechnischen Maßstab. Evtl. noch im Strom **3** (**31, 32**) vorhandene Anteile an Flüssigkeitstropfen sind ganz überwiegend bis vollständig schwerflüchtigen Begleitkomponenten im DNT technischer Reinheit geschuldet. Der Anteil an unverdampften Tröpfchen in Strom **3 (31**, **32**) ist daher wesentlich von der Reinheit des eingesetzten DNT abhängig. Das im erfindunggemäßen Verfahren als DNT-Quelle des Stroms **1** (**11, 12**) bevorzugt eingesetzte "technische" DNT umfasst bevorzugt:
99,00 Massen-% bis 99,94 Massen-% DNT, wobei 2,4- und 2,6-DNT den Hauptanteil ausmachen (> 95 % des in **1** enthaltenen DNT);
> 5 ppm Nitrokresole;
> 20 ppm Trinitrotoluol;
> 500 ppm sonstige Verunreinigungen umfassend, bevorzugt bestehend aus Wasser, Mononitrotoluol, Schwefelsäure und Salzen der Schwefelsäure,
jeweils bezogen auf die Gesamtmasse des technischen DNT **1** (**11, 12**).

Die eingesetzte Verdampfungsapparatur (**1000, 1010, 1020**) muss eine möglichst vollständige Verdampfung des DNT bei gleichzeitig kurzer Verweilzeit und minimaler thermischer Belastung des flüssigen DNT gewährleisten. Hierzu eignen sich erfindungsgemäß solche Apparaturen, in denen der DNT-haltige Strom 1 in einen heißen Wasserstoff-haltigen Trägergasstrom **2** (**21, 22**) mittels wenigstens einer Sprüh-Vorrichtung (bevorzugt eine Düse) eingesprüht wird ("Sprühverdampfer"). Dabei liegt das Verhältnis der Molenströme von **1** zu **2** (**21, 22**) bevorzugt so, dass der Anteil von DNT in **3** (**31, 32**) von 0,1 Mol-% bis 10 Mol-%, besonders bevorzugt von 0,8 Mol-% bis 2,0 Mol-%, jeweils bezogen auf die Gesamtstoffmenge aller in Strom **3** (**31, 32**) enthaltenen Verbindungen, beträgt. Die Temperatur des Trägergasstroms ist so zu wählen, dass möglichst viel DNT verdampft werden kann, d. h. der DNT-Partialdruck möglichst nah am Sättigungsdruck bei der gegebenen Austrittstemperatur aus dem Verdampfer liegt, und gleichzeitig im stromabwärts angeordneten Reaktionsraum bei vollständigem DNT-Umsatz die maximal erlaubte Temperatur von 300 °C nicht überschritten wird. Daraus ergibt sich eine geeignete Temperatur des wasserstoffhaltigen Trägergases **2** am Eintritt der Verdampfungsapparatur von 140 °C bis 300 °C, bevorzugt von 180 °C bis 240 °C. Um eine möglichst schnelle (innerhalb von 0,010 s bis 100 s, bevorzugt innerhalb von 0,010 s 3,0 Sekunden) und vollständige (mindestens 95,0 Massen-%, bevorzugt mindestens 99,5 Massen-% allen in **1** (**11, 12**) vorhandenenen DNT's) Verdampfung des DNT zu erreichen, ist ein DNT-Sprühstrom **1** (**11, 12**) mit möglichst kleiner Tropfengröße (mittlerer Tropfendurchmesser d bevorzugt zwischen 20 µm und 200 µm) und gleichmäßiger Tropfengrößenverteilung zu erzeugen, wie es mit gängigen Ein- und insbesondere Zweistoffdüsen möglich ist.

Geeignete Einstoffdüsen sind beispielsweise die in Wozniak, "Zerstäubungstechnik", Springer 2003 (insbesondere Kapitel 5.1, 5.3, 5.4 und 5.5) und Richter "Zerstäuben von Flüssigkeiten", expert Verlag, Renningen, 2004 (insbesondere Kapitel 3.4, 4.3, 4.4 und 5.7), beschriebenen. Tangential-Hohlkegeldüsen beispielsweise eignen sich auf Grund der relativ kleinen erzeugten Tropfendurchmesser und ihrer Verstopfungsunempfindlichkeit. Für kleinere Mengenströme und noch kleinere Tropfendurchmesser eignen sich auch Ultraschallzerstäuberdüsen, auch wegen ihres variablen Betriebsbereichs.

Die oben genannten Anforderungen an Tropfengröße und Tropfengrößenverteilung lassen sich jedoch am Einfachsten mit Zweistoffdüsen verwirklichen. In einer ganz besonders bevorzugten Ausführungsform betrifft die Erfindung daher ein Verfahren, bei dem das Einsprühen des Stromes **1** (**11, 12**) in den Trägergasstrom **2** (**21, 22**) in Schritt (I) mittels wenigstens einer Zweistoffdüse (**7000**) erfolgt, durch die zusätzlich zu **1** (**11, 12**) ein Strom eines Zerstäubungsgases **9** geleitet wird, das gegenüber dem in der Umgebung auf der Düsenaustrittsseite herrschenden absoluten Druck unter einem 1,0 bar bis 20 bar, bevorzugt 3,0 bar bis 9,0 bar, höheren Druck steht und bevorzugt die gleiche Temperatur hat wie Strom **2.** Geeignete Zweistoffdüsen sind beispielsweise beschrieben in Wozniak, "Zerstäubungstechnik", Springer 2003 (insbesondere Kapitel 5.2) und Richter "Zerstäuben von Flüssigkeiten", expert Verlag, Renningen, 2004 (insbesondere Kapitel 6.5). Die Vermischung von aus **1** (**11, 12**) und dem Zerstäubungsgasstrom (**9**) erfolgt je nach Konstruktion der Zweitstoffdüse **7000** entweder in der Düse ("innenmischende Düse") oder bei Austritt der einzelnen Ströme aus der Düse ("außenmischende Düse"). Im ersteren Fall wird in der Düse **7000** ein zweiphasiges Gemisch aus **1** (**11, 12**) und dem Zerstäubungsgasstrom (**9**) erzeugt, das in den Trägergasstrom **2** (**21, 22**) eingesprüht wird. Im Letzteren Fall werden die Ströme enthaltend DNT **1** (**11, 12**) und Zerstäubungsgas (**9**) getrennt voneinander durch Kanäle in der Düse in die Trägergasatmosphäre **2** (**21, 22**) eingesprüht. Bevorzugt werden innenmischende Zweistoffdüsen eingesetzt, bei denen die Ströme **1** (**11, 12**) und **9** innerhalb der Düse aufeinandertreffen und als zweiphasiges Gemisch aus der Düse austreten.

In bevorzugten Ausführungsformen betrifft die Erfindung ein Verfahren, bei dem als Zerstäubungsgas **9** Wasserdampf, Stickstoff, Wasserstoff, ein Teil der Wasserstoff umfassenden Gasphase **7**, ein Teil des wasserstoffhaltigen Trägergasstromes (**2, 21, 22**) oder eine Mischung aus zwei oder mehr der vorgenannten Gase eingesetzt wird. Ganz besonders bevorzugt wird Wasserstoff (d. h. Frischwasserstoff **200**, zu unterscheiden vom Wasserstoff-haltigen Strom **2**(**21**, **22**) als Zerstäubungsgas **9** eingesetzt. Bei mehreren in Serie geschalteten Reaktoren wird Strom **200** auf die verschiedenen Reaktoren aufgeteilt (**210**, **220**,...),. So kann im kontinuierlichen Betrieb bei möglichst vollständiger Rezyklierung des Prozessgases 7 auf einfache und zweckmäßige Weise der in der Hydrierung verbrauchte Wasserstoff ersetzt werden. Das Massenverhältnis von Strom **9** zu Strom **1** beträgt bevorzugt von 0,01 bis 1, besonders bevorzugt von 0,05 bis 0,2. Die Druckverluste über die Düse betragen bevorzugt 1,0 bar bis 20 bar, besonders bevorzugt 3,0 bar bis 9,0 bar, für Strom **9** und bevorzugt 0,1 bar bis 20 bar, besonders bevorzugt 3,0 bar bis 9,0 bar, für Strom **1.**

In **Schritt (II)** wird, u. a. wegen der höheren thermischen Empfindlichkeit in der flüssigen Phase, der bereits im Wesentlichen gasförmige Strom **3** (**31, 32**) noch weiter von Flüssigkeitströpfchen befreit. Dies geschieht bevorzugt durch eine oder mehrere der folgenden Maßnahmen:
a) Abtrennung der übrigen Tropfen aus Strom **3** (**31, 32**), z. B. mittels einer geeigneten Abscheideeinheit. Eine solche Abscheideeinheit kann auch im Reaktor selbst eingebaut sein. Sie ist dann dem eigentlichen Reaktionsraum vorgelagert. Geeignete Abscheideeinheiten sind beispielsweise dem Fachmann bekannte Filter, Gestricke, Umlenkabscheider, Zyklone und Tropfenabscheider. Die Abscheideeinheiten werden entweder so dimensioniert, dass ihre Funktion für die Dauer eines gewöhnlichen Produktionszyklus gewährleistet ist, wonach sie dann gereinigt bzw. in dem Fachmann bekannter Weise (z. B. durch Abbrennen) regeneriert werden, oder es werden mehrere Abscheideeinheiten parallel geschaltet, von denen eine in Betrieb ist während die anderen gereinigt bzw. regeneriert werden.
b) Gezielte kontinuierliche oder diskontinuierliche Zersetzung der akkumulierten Bestandteile zu ungefährlichen Spezies, z. B. durch geeigneten Energieeintrag und kontrolliertes Hervorrufen der thermischen Zersetzung.
Dabei ist a) gegenüber b) bevorzugt.

Da DNT und TDA miteinander reagieren können, müssen die Reaktionsbedingungen in **Schritt (III)** so gewählt werden, dass innerhalb kurzer Zeit ein möglichst vollständiger Umsatz des DNT gewährleistet ist. Hier sind vor allem die oben genannten Bedingungen im Hinblick auf Druck, Temperatur und das molare Verhältnis von Wasserstoff zu DNT von Bedeutung. Als Katalysatoren eignen sich grundsätzlich die dem Fachmann für die DNT-Hydrierung bekannten Katalysatoren, sofern sie eine rasche Umsetzung des DNT ermöglichen. Erfindungsgemäß werden Feststoffkatalysatoren eingesetzt. Grundsätzlich sind alle dem Fachmann bekannten Feststoffkatalysatoren für die Hydrierung aromatischer Nitroverbindungen geeignet. Solche Katalysatoren sind in vielen Publikationen beschrieben und umfassen als hydrieraktive Elemente Pd, Pt, Ru, Fe, Co, Ni, Mn, Re, Cr, Mo, V, Pb, Ti, Sn, Dy, Zn, Cd, Ba, Cu, Ag, Au, und deren Verbindungen, zum Teil als Oxide, Sulfide oder Selenide und auch in Form einer Raney-Legierung sowie auf inerten Trägermaterialien, wie beispielsweise Al₂O₃, Fe₂O₃/Al₂O₃, SiO₂, Silikaten, Kohle, Graphit, TiO₂, Cr₂O₃. Mischoxide der genannten Elemente sind ebenfalls denkbar.

Bevorzugt wird ein Katalysator (**100, 110, 120**) umfassend einen keramischen Träger, bevorzugt Al₂O₃, besonders bevorzugt α-Al₂O₃, ganz besonders bevorzugt α-Al₂O₃ mit einer BET-Oberfläche von weniger als 40 m²/g, bevorzugt weniger als 20 m²/g, besonders bevorzugt weniger als 10 m²/g, und
(a) 1,0 g bis 100 g, bevorzugt 1,0 g bis 50 g, wenigstens eines Metalls der Gruppen 8 bis 12 des Periodensystems der Elemente (Nummerierung gemäß IUPAC-Empfehlung von 1986), bevorzugt Pd, Pt,
(b) 1,0 g bis 100 g, bevorzugt 1,0 g bis 50 g, wenigstens eines Metalls der Gruppen 4 bis 6 und 12 des Periodensystems der Elemente, bevorzugt Ti, V, Nb, Ta, Cr, Mo, W, und
(c) 1,0 g bis 100 g, bevorzugt 1,0 g bis 20 g, wenigstens eines Metalls der Gruppen 14 und 15 des Periodensystems der Elemente, bevorzugt Pb, Bi,
pro Liter Schüttvolumen des keramischen Trägers,
eingesetzt.

Weitere bevorzugte Katalysatoren umfassen als hydrieraktive Elemente
Pd und Rh oder
Ag und Rh
jeweils auf einem inerten Träger, bevorzugt Al₂O₃, besonders bevorzugt α-Al₂O₃.

Die Reaktion an einem Feststoffkatalysator kann weitgehend isotherm (d. h. unter Abfuhr der Reaktionswärme), z. B. in einem Rohrbündelreaktor oder Wirbelschichtreaktor durchgeführt werden. Geeignete Apparate sind in Perry's Chemical Engineers' Handbook, 8. Auflage, Kapitel 19, 2007, und 7. Auflage, Kapitel 23, 1999, Mcgraw-Hill Professional, beschrieben. Es ist auch möglich, die Reaktion adiabat, z. B. in einem Festbettreaktor (wie in DE 10 2006 035 203 A1, insbesondere in den Abschnitten [0006], [0020] und [0030] bis [0032] beschrieben) durchzuführen. In einem adiabaten Reaktor wird bevorzugt ein adiabater Temperatursprung von 50 K bis 150 K eingestellt. Zur Einhaltung dieses Temperatursprungs ist der Gasstrom **4** entsprechend einzustellen (bspw. durch einen hinreichend großen Wasserstoffüberschuss). Dem Fachmann ist bekannt, wie die erforderliche Wahl des Gasstroms **4** zu berechnen ist. Eine Kombination der verschiedenen Fahrweisen ist ebenfalls denkbar.

Die Abfuhr der Reaktionswärme erfolgt bei isotherm betriebenen Reaktoren im Reaktor integriert (z. B. bei Rohrbündelreaktoren mit Kühlkreislauf (z. B. Öl, Wasser oder Salzschmelze) zur Abfuhr der Reaktionswärme). Adiabat betriebenen Reaktoren sind bevorzugt Vorrichtungen zur Wärmeabfuhr (z. B. Wärmeaustauscher) nachgeschaltet. Das Temperaturniveau erlaubt die Nutzung der abzuführenden Wärme zur Erwärmung geeigneter Wärmeträger, z. B. zur Erzeugung von Wasserdampf einer möglichst hohen Druckstufe, bevorzugt 3 bis 10 bar (absolut).

Es können mehrere Reaktionsstufen mit oder ohne Zwischeneinspeisung eines oder mehrerer Edukte in Serie geschaltet werden. Wird in jeden Reaktor DNT eingespeist, verfügt bevorzugt jeder Reaktor über eine eigene DNT-Verdampfungsstufe. Zweckmäßigerweise wird das aus der letzten Reaktionsstufe austretende Gas nach weitgehender Entfernung der Reaktionsprodukte und Ausschleusung unerwünschter Komponenten in die Verdampfungs- und/oder Reaktionsstufen zurückgeführt, um im Überschuss eingesetzte Edukte (d. h. den Wasserstoff) zu nutzen, die Verdampfung zu erleichtern und den Temperatursprung in den Reaktoren zu senken.

Das Hintereinanderschalten mehrerer Reaktionsstufen ist insbesondere bei adiabater Reaktionsführung vorteilhaft, da die pro Stufe umsetzbare Menge an DNT durch den niedrigen Dampfdruck einerseits und den adiabaten Temperatursprung andererseits begrenzt sein kann. Kommt ein weitgehend isothermer Reaktor zum Einsatz, kann der Einsatz einer vorgeschalteten adiabaten Reaktionsstufe ohne oder geringerer Zwischenkühlung vorteilhaft sein, um den Gasstrom vor der Verdampfung mittels der Reaktionswärme vorzuwärmen, sodass die DNT-Verdampfung erleichtert wird. Eine besonders effiziente Ausführung des Verfahrens stellt der abwechselnde Einsatz adiabater und isothermer Reaktionsstufen dar, da die besonders hohe Austrittstemperatur der adiabaten Reaktionsstufen zur Verdampfung großer Mengen DNT für den Umsatz in den isothermen Stufen genutzt werden kann. Es ist auch denkbar, die Schritte (I) bis (III) in einem Apparat zu integrieren.

Die Hydrierung in Schritt (III) wird vorteilhafterweise bei einem absoluten Druck von 1,0 bar bis 20 bar, bevorzugt von 3,0 bar bis 6,0 bar durchgeführt. Niedrigere Drücke erleichtern die DNT-Verdampfung und Wärmerückgewinnung in der Produktabtrennung, führen aber zu größeren Apparatedimensionen und höherem Energieeinsatz in der Verdichtung. Insbesondere ist der Rückführstrom im industriellen Maßstab durch maximal verfügbare Apparategrößen begrenzt, so dass ein niedrigerer Prozessdruck bei gleicher Anlagenkapazität und gleichem maximalen Rückführ-Volumenstrom mehr Reaktionsstufen bedingt.

Wasserdampf oder Stickstoff können zwecks Erhöhung der mittleren Molmasse des zu verdichtenden Kreisgases (gemittelt über alle vorhandenen Komponenten) zur günstigeren Verdichtung in Verdampfung und Reaktion gezielt zugeführt oder durch gezielte Ausschleusung in den gewünschten Konzentrationen im Prozess belassen werden.

Die Reaktionsprodukte Toluylendiamin (TDA) und Wasser werden in **Schritt (IV)** durch Kondensation selektiv aus dem Toluylendiamin enthaltenden Gasstrom entfernt. Bei mehreren in Serie geschalteten Reaktoren kann dies entweder nach jedem Reaktor oder bevorzugt nur nach dem letzten Reaktor geschehen. Geeignete Apparate hierfür sind dem Fachmann bekannt und sind z. B. Luftkühler oder Rohrbündelwärmeaustauscher. Die Kondensation erfolgt bevorzugt fraktionierend in mehreren hintereinandergeschalteten Kondensatoren (**4010, 4020, 4030**) mit sukkzesive sinkender Kondensationstemperatur, wobei die Gasphase eines Kondensators in den folgenden Kondensator geleitet wird. In einer bevorzugten Ausführungsform erfolgt die Kondensation so, dass zwei bis fünf, besonders bevorzugt vier, Kondensatfraktionen erhalten werden. In einer Ausführungsform mit fünf Kondensatfraktionen **6a, 6b, 6c, 6d** und **6e** kann die Kondensationstemperatur beispielsweise sukzessive wie folgt gesenkt werden:
**6a:** Kondensation bei 175 °C bis 195 °C,
**6b:** Kondensation bei 145 °C bis 165 °C,
**6c:** Kondensation bei 129 °C bis 149 °C,
**6d:** Kondensation bei 85 °C bis 105 °C
**6e:** Kondensation bei 30°C bis 50 °C.

Die auf diese Weise erhaltenen verschiedenen Kondensatströme (**6a, 6b, 6c**, ...) werden bevorzugt getrennt voneinander in verschiedene Stellen einer sich anschließenden Destillationssequenz geleitet. Diese Destillationssequenz kann aus gewöhnlichen Destillationskolonnen (oder einer einzelnen Destillationskolonne) bestehen, wie sie dem Fachmann geläufig sind (ist). In besonders bevorzugten Ausführungsformen umfasst die Destillationssequenz
1. wärmeintegrierte Konfigurationen wie bspw. Kolonnen mit Wärmepumpen oder sog. HIDIC's ("Heat integrated distillation columns") oder
2. thermisch gekoppelte Kolonnen wie bspw. Kolonnen mit Seitenstromstripp- oder Rektifikationskolonnen, Seitenstromverdampfer oder Vortrennkolonnen oder Vorverdampfer, wie bspw. sog. Petlyuk- oder Kaibel-Konfigurationen, oder Trennwandkolonnen.

Darüber hinaus können die Destillationskolonnen eine Anzahl Flüssigkeits-Umpumpkreisläufe aufweisen.

Werden mehrere Kondensatfraktionen **6a, 6b,** etc. derselben Destillationskolonne zugeführt, so ist es bevorzugt, die bei höherer Temperatur erhaltenen Fraktionen oberhalb der bei geringerer Temperatur erhaltenen Fraktionen der Destillationskolonne zuzuführen.

Die genannten Destillationssequenzen sind erheblich kostengünstiger zu installieren und zu betreiben als eine TDA-Destillationssequenz nach dem Stand der Technik mit bis zu sechs Destillationskolonnen.

Aus dem verbleibenden nicht kondensierbaren Gasstrom wird bevorzugt ein Teilstrom ausgeschleust, um die Akkumulation flüchtiger unerwünschter Komponenten im Prozess zu vermeiden. Im Anschluss wird bevorzugt der Druck erhöht und der verdichtete Strom wieder in den Prozess zurückgeführt (Wasserstoff-haltiger Gasstrom **7**).

Die in Schritt (IV) erhaltene Wasserstoff umfassende Gasphase **7** wird in **Schritt (V)** in die erste Verdampfungsapparatur (**1000, 1010**) zurückgeführt. In einer bevorzugten Ausführungsform wird die Wasserstoff umfassende Gasphase **7** verwendet, um den wasserstoffhaltigen Trägergasstrom **2** bereitzustellen. Dies kann so geschehen, dass Frischwasserstoff **200** mit dem DNT-haltigen Strom **1** vermischt (bevorzugt in einer Zweistoffdüse) wird, ehe der so erhaltene Mischstrom in den Trägergasstrom **2** (in dieser Ausführungsform identisch mit dem rezyklierten Gasstrom **7**) eingesprüht wird (in FIG. 1 mit gestrichelten Linien gezeigt). Alternativ ist auch denkbar (wie in FIG. 1 mit durchgezogenen Linien gezeigt), Frischwasserstoff (**200**) mit dem rezyklierten Prozessgasstrom **7** zum Trägergasstrom **2** zu vereinigen, ehe der DNT-haltige Gasstrom **1** eingesprüht wird. Im Falle mehrerer in Serie geschalteter Reaktoren wird der Strom 7 unmittelbar nur für die Bereitstellung des Trägergasstromes **2** für den ersten Reaktor verwendet. Der Trägergasstrom für die folgenden Reaktoren ist der Toluylendiamin enthaltende Gasstrom (**51**), bzw., falls dieser, was bevorzugt ist, mit Frischwasserstoff (**200**) angereichert wird, wie in FIG. 3 gezeigt, der Strom **22,** welcher natürlich die Bestandteile des Stroms **7** enthält.

Die in Schritt (IV) erhaltene Flüssigphase **6** (bzw. die Flüssigphasen **6a, 6b, 6c** etc.) enthält neben dem Zielprodukt Toluylendiamin hauptsächlich Wasser sowie in untergeordneten Mengenanteilen Nebenkomponenten. Strom **6** (bzw. die Flüssigphasen **6a, 6b, 6c** etc.) wird bevorzugt in einem weiteren **Schritt (VI)** durch an sich bekannte Verfahren umfassend die Destillation des rohen TDA aufgearbeitet, um Rein-Toluylendiamin zu gewinnen. Geeignete Verfahren sind beispielsweise in US 6,359,177 und US 7,307,190 beschrieben. Durch eine geeignete Vorfraktionierung in Schritt (IV) kann diese Aufarbeitung noch deutlich vereinfacht werden.

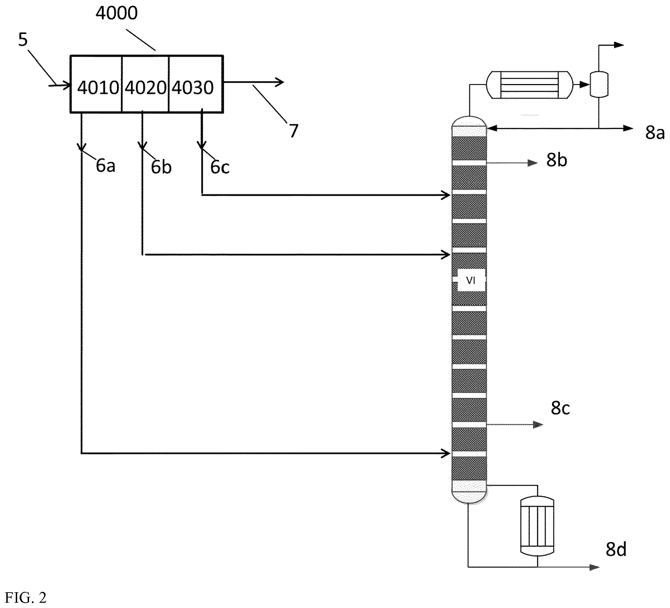

Nachstehend werden verschiedene Ausführungsformen der Erfindung anhand der Zeichnungen näher erläutert:
**FIG. 1** zeigt in stark vereinfachter Form eine grundlegende Ausführungsform des erfindungsgemäßen Verfahrens mit nur einem Reaktor **3100.** Auf die Darstellung von Details wie bspw. Verdichtern wurde aus Gründen der Übersichtlichkeit verzichtet.
   DNT technischer Reinheit (**1**) wird in einer Verdampfungsapparatur **1000** durch Einsprühen in den wasserstoffhaltigen Trägergasstrom **2** in die Gasphase überführt (Schritt (I), durchgezogene Linien). Es ist auch denkbar, den Prozessgasstrom **7** als wasserstoffhaltigen Trägergasstrom **2** zu verwenden und Frischwasserstoff **200** vor Eintritt in die Verdampfungsapparatur mit dem DNT-Strom **1** zu vermischen (gestrichelte Linien). Vor Eintritt in den Reaktor wird der Strom **3** in einer Vorrichtung **2000** von letzten Flüssigkeitströpfchen befreit (Schritt (II)). Der dabei erhaltene flüssige Strom **8** wird zur Vermeidung unerwünschter Reaktionen entweder rasch weiter abgekühlt oder kontrolliert zersetzt (z. B. durch Mikrowellenbestrahlung oder anderweitig herbeigeführte kontrollierte thermische Zersetzung; nicht gezeigt in der Abbildung). Der Gasstrom **4** wird in den Reaktor **3100** geleitet. Im Reaktionsraum **3000** findet die Hydrierung in Gegenwart des Katalysators **100** statt (Schritt (III)). Der den Reaktor verlassende gasförmige Produktstrom **5** wird im Wärmeaustauscher **5000** durch Wärmeaustausch mit Strom **7** gekühlt und in der Kondensationsapparatur **4000** kondensiert, wobei eine Toluylendiamin umfassende Flüssigphase **6** und eine Wasserstoff umfassende Gasphase **7** erhalten werden. Strom **7** wird, ggf. nach Ausschleusung eines kleinen Anteils (nicht gezeigt in der Abbildung) zur Verhinderung der Anreicherung unerwünschter Gase, mit dem Frischwasserstoff **200** vermischt, um den wasserstoffhaltigen Trägergasstrom **2** zu erhalten. Strom **7** wird im Wärmeaustauscher **5000** durch Wärmeaustausch mit Strom **5** vorgewärmt. Im Wärmeaustauscher **5000** kann bereits eine partielle Kondensation des Stroms **5** erfolgen. Im Allgemeinen wird diese jedoch nicht vollständig genug sein, sodass ein Kondensator **4000** nachgeschaltet wird.
**FIG. 1a** zeigt in einem Ausschnitt des Gesamtverfahrens der in FIG. 1 gestrichelt dargestellten Ausführungsform eine bevorzugte Ausgestaltung dieser Ausführungsform, in welcher eine Zweistoffdüse **7000** eingesetzt wird. Frischwasserstoff **200** dient gleichzeitig als Zerstäubungsgas **9.** Die Zweistoffdüse mündet direkt in die Verdampfungsapparatur **1000.** Eine mögliche Ausgestaltung einer Zweistoffdüse **7000** als innenmischende Düse zeigt **FIG. 1b****.**
   Es ist bevorzugt, die Kondensation in Apparat **4000** so durchzuführen, dass bereits auf dieser Stufe eine Vortrennung des rohen TDA stattfindet, welche die nachfolgende Aufarbeitung erleichtert (**FIG. 2**). Hierzu wird das gasförmig anfallende Produkt **5** durch eine fraktionierende Kondensation in hintereinander geschalteten Kondensatoren (**4010, 4020, 4030**) mit sukzessive sinkender Temperatur in mehrere Kondensatfraktionen **6a, 6b, 6c,** etc. aufgetrennt. Die in der Zeichnung gezeigte Ausführungsform mit drei Kondensatströmen ist exemplarisch zu verstehen. Jede der so erhaltenen Kondensatfraktionen wird der nachfolgenden Destillationssequenz (**Schritt (VI)**) an einer anderen, genau auf diese Fraktion abgestimmten Stelle der Destillationskolonne **6000** zugeführt. Auf diese Weise werden verschiedene Toluylendiamin-Fraktionen **8a, 8b, 8c** und **8d** erhalten, die sich in ihrer Isomerenzusammensetzung und im Nebenkomponentengehalt unterscheiden.
**FIG. 3** zeigt eine Anordnung, in der anstelle eines Reaktors **3100** zwei adiabat betriebene Reaktoren **3110** und **3120** mit zwei Verdampfungsapparaturen **1010** und **1020** in Serie geschaltet sind. Die Darstellung von zwei Reaktoren ist rein exemplarisch; die tatsächliche Anzahl der zu wählenden Reaktoren hängt von vielen Faktoren wie der angestrebten Produktionskapazität, dem Kreisgasvolumen u. a. ab. Bevorzugt sind 8 bis 12 Reaktoren in Serie geschaltet. Die in FIG. 1 mit gestrichelten Linien dargestellte Reaktionsführung ist hier natürlich ebenfalls anwendbar und wurde nur aus Gründen der Übersichtlichkeit nicht eingezeichnet.

DNT technischer Reinheit (**1**) wird in einer Verdampfungsapparatur **1010** bzw. **1020** durch Einsprühen in den wasserstoffhaltigen Trägergasstrom **21** bzw. **22** in die Gasphase überführt (Schritt (I)). Vor Eintritt in den jeweiligen Reaktor wird der Strom **31** bzw. **32** in einer Vorrichtung **2010** bzw. **2020** weiter von Flüssigkeitströpfchen befreit (Schritt (II)) und so ein Gasstrom **41** bzw. **42** erhalten. In den Reaktoren **3110** und **3120** findet die Hydrierung unter adiabaten Bedingungen statt (Schritt (III)). Die Reaktionswärme spiegelt sich, bis auf unvermeidbare geringfügige Wärmeverluste, quantitativ in einer Temperaturzunahme des Gasstroms wider (adiabater Temperatursprung). Der den ersten Reaktor verlassende gasförmige Produktstrom **51** wird unter Dampfgewinnung in einem nachgeschalteten Wärmeaustauscher **5010** auf die Eintrittstemperatur der folgenden Stufe abgekühlt. Nach Durchlaufen des letzten Wärmeaustauschers **5020** durchläuft das Produkt eine mehrstufige Kondensation und Phasentrennung in **4000** (Schritt IV). Die dabei erhaltenen Ströme **6a, 6b** und **6c** werden in Schritt (VI) (in der Abbildung nicht gezeigt) zu Rein-TDA aufgearbeitet. Die erhaltene Gasphase wird, nach Wärmeaustausch mit Strom **52** und ggf. nach Ausschleusung eines kleinen Teils als Purge-Strom (in der Abbildung nicht gezeigt), als Strom **7** in den Prozess zurückgeführt (Schritt (V)).

Das erfindungsgemäße Verfahren kann in verschiedenen Ausführungsformen durchgeführt werden. Die Reaktion im Reaktionsraum **3000** (**3010, 3020**) kann, wie bereits erwähnt, adiabat oder isotherm durchgeführt werden. Der Katalysator 100 (110, 120) kann in Form eines Festbetts oder fluidisiert vorliegen. Das Verfahren kann darüber hinaus mehrstufig (d. h. in mehreren Reaktionsräumen **3010, 3020**, ...) mit mehrfacher Zugabe von frischem DNT durchgeführt werden, wobei zwei bis 10 Stufen bevorzugt sind. Dabei kann die Art des Reaktionsraums **3000** von Stufe zu Stufe variieren. Es ist bspw. eine Ausführungsform denkbar, in der sich adiabat und isotherm betriebene Reaktionsräume abwechseln (**3010**: adiabat, **3020**: isotherm, **3030**: adiabat, usw.). Es können auch Festbettreaktoren und Wirbelschichtreaktoren in einer Produktionsanlage miteinander kombiniert werden, bspw. abwechselnd.

Darüber hinaus können zwischen den einzelnen Stufen Vorrichtungen zur Wärmeabfuhr (Wärmeaustauscher) vorgesehen sein. Bevorzugt werden solche Vorrichtungen zur Wärmeabfuhr den adiabat betriebenen Reaktionsräumen nachgeschaltet. Bei isotherm betriebenen Reaktionsräumen erfolgt die Wärmeabfuhr im Reaktionsraum selbst. Es ist ebenfalls möglich, die Reaktionswärme der vorangegangenen Stufe teilweise bis vollständig für die (naturgemäß endotherme) Verdampfung von frischem DNT in jeder Stufe zu verwenden und so direkt nutzbar zu machen.

### Beispiele

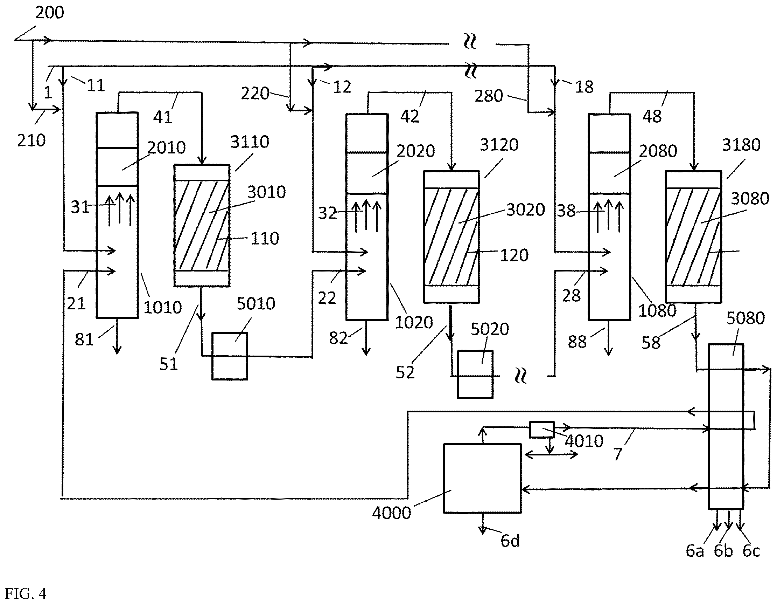

### Beispiel 1 (Verfahrenssimulation mit Aspen Plus® für einen Prozess mit acht in Serie geschalteten Reaktoren; FIG. 4):

39,8 Tonnen DNT (**1**) wurden pro Stunde bei 70 °C bis 100 °C geschmolzen, auf einen absoluten Druck von 10 bis 20 bar verdichtet und in acht Ströme (**11, 12, ..., 18**) aufgeteilt. 2959 kg/h Frischwasserstoff (**200**) wurden bei dem gleichen absoluten Druck zugeführt und ebenfalls in acht Ströme (**210, 220, ..., 280**) aufgeteilt. DNT- (**11, 12, ..., 18**) und Frischwasserstoffströme (**210, 220,..., 280**) wurden jeweils in Zweistoffdüsen (nicht abgebildet) miteinander vermischt. Die resultierenden Mischströme wurden Sprühverdampfern (**1010, 1020, ..., 1080**) zugeführt. Im ersten Sprühverdampfer wurde Kreisgas (**7**; ca. 100.000 m³/h) unter einem absoluten Druck von 4,2 bar als Trägergasstrom (**21**) verwendet, in den das DNT/Frischwasserstoffgemisch (**11**+**210**) eingesprüht wurde. Der resultierende überwiegend gasförmige Mischstrom (**31**) wurde durch einen Tropfenabscheider (**2010**) geführt. Die Verweilzeit des DNT vom Eintritt in den Sprühverdampder bis Eintritt in den Tropfenabscheider betrug dabei 0,25-0,35 sec. In der gezeigten Ausführungsform ist die Vorrichtung zur Tropfenabscheidung in die Verdampfungsapparatur integriert. Nicht verdampfbare Anteile wurden als flüssiger Strom (**81**) am Sumpf des Sprühverdampfers (**1010**) abgezogen. Alle so erhaltenen Flüssigströme unverdampfbarer Anteile (**81, 82, ..., 88**) wurden rasch auf eine Temperatur unter 40°C abgekühlt und sicher entsorgt (nicht gezeigt in der Abbildung). Der Gasstrom (**41**) aus dem Sprühverdampfer wurde in den Reaktor **3110** geführt. Im Reaktionsraum **3010** wurde DNT unter adiabaten Bedingungen zu TDA umgesetzt. Der aus dem Reaktor austretende TDA-haltige Kreisgasstrom (**51**) wurde in einem Wärmeaustauscher (**5010**) unter Gewinnung von 6-bar-Dampf auf eine Temperatur von 180°C gekühlt und dem nächsten Sprühverdampfer (**1020**) als Trägergasstrom (**22**) für das DNT/Frischwasserstoffgemisch (**12**+**220**) zugeführt. Der erhaltene Mischstrom (**32**) wird wie zuvor beschrieben von Tropfen befreit und im Reaktor **3120** zu TDA umgesetzt. Die weitere Umsetzung in den Reaktoren **3130** bis **3180** erfolgte analog mit geringfügig abweichenden Temperaturen.

Der nach Durchlaufen des letzten Reaktors (**3180**) erhaltene Produktgasstrom (**58**) wurde durch mehrere Wärmeaustauscher geführt (vereinfacht als **5080** dargestellt), in denen bereits eine fraktionierende partielle Kondensation zu flüssigen TDA-haltigen Strömen (**6a, 6b, 6c**) erfolgte. Der verbleibende Gasstrom wurde in einem Wäscher (**4000**) partiell zur TDA-haltigen Flüssigsphase **6d** verflüssigt. Das Kopfprodukt des Wäschers (**4000**) wurde durch einen Kondensator (**4010**) geleitet, die dort erhaltene Flüssigphase, die überwiegend aus Wasser und untergeordneten Anteilen von niedrig siedenden Nebenprodukten und Spuren von TDA bestand, partiell in den Wäscher zurückgeführt und partiell ausgeschleust. Dem aus dem Kondensator (**4010**) austretenden Kreisgasstrom (**7**) wurde ein kleiner Purge-Strom entnommen, und der verbleibende Kreisgasstrom (**7**) wurde verdichtet (beides nicht gezeigt in der Abbildung). Nach Aufheizung durch indirekten Wärmeaustausch mit dem heißen TDA-Gasstrom **58** in **5080** wurde der Kreisgasstrom (**7**) als Trägergasstrom (**21 = 7**) der Verdampfung des DNT/Frischwasserstoffgemisches (**11**+**210**) im Sprühverdampfer (**1010**) zugeführt.

Die Kondensation der einzelnen TDA-Fraktionen erfolgte bei folgenden Temperaturen:
**6a:** 185 °C
**6b**: 155 °C
**6c:** 135 °C
**6d:** 95 °C

Diese vier Ströme wurden getrennt voneinander einer Destillationskolonne mit Umlaufverdampfer, Seitenabzug und Kopfkondensator zugeführt (nicht gezeigt in FIG. 4), wobei der Strom **6a** 5 theoretische Stufen oberhalb des Sumpfes in die Kolonne eingespeist wurde. Strom **6b** wurde oberhalb von **6a,** Strom **6c** oberhalb von **6b** und Strom **6d** oberhalb von 6c eingespeist. Der absolute Druck am Kopf der Destillationskolonne betrug 60 mbar. Es wurden vier Produktströme entnommen:
Gasförmiges Kopfprodukt überwiegend umfassend Wasser und Leichtsieder;
flüssiges Kopfprodukt überwiegend umfassend o-TDA;
ein als Seitenstrom entnommenes Produkt überwiegend umfassend m-TDA (25 Tonnen pro Stunde);
Sumpfprodukt überwiegend umfassend Schwersieder.

Der Energiebedarf des Umlaufverdampfers betrug 5,5 MW bei 202 °C.

Für eine solche Destillationsleistung ist nach dem Stand der Technik der Einsatz mehrerer Destillationskolonnen erforderlich. Dies wird in der Fachliteratur deutlich; siehe exemplarisch Kirk-Othmer, Encyclopedia of Chemical Technology, John Wiley & Sons Inc; 5. Auflage (31. Januar 2004), Vol. 2, Seite 485, Online-ISBN: 9780471238966, wo der Einsatz von drei Destillationskolonnen für die Wasser- und Isomerentrennung beschrieben wird. EP1935871A2 beschreibt, dass durch eine geeignete Energiesparverschaltung mit der Reaktionssektion umgerechnet 10,9 MW Fremddampf für die Wasserabtrennung zur Gewinnung von 25 t/h m-TDA-Produktstrom benötigt werden. Gemäß US7307190B2 lässt sich die anschließende Isomerentrennung energiesparend in einer Trennwandkolonne bewerkstelligen, wofür eine Verdampferleistung von umgerechnet 5,6 MW für die Gewinnung von 25 t/h m-TDA-Produktstrom benötigt wird. Nach dem Stand der Technik werden für die gesamte Reaktionsgemischauftrennung demnach in Summe mindestens 15,5 MW Verdampferleistung in mindestens zwei Kolonnen benötigt, im Vergleich zu 5,5 MW gemäß dem erfindungsgemäßen Verfahren. Darüber hinaus ist die erfindungsgemäße Destillation in einer Kolonne apparativ kostengünstiger herzustellen und einfacher zu betreiben als die zwei-Kolonnen-Destillationssequenz nach dem Stand der Technik, bestehend aus einer Kolonne mit Wärmeintegration der Reaktionssektion und aus einer Trennwandkolonne.

## Patentansprüche

1. Kontinuierliches Verfahren zur Herstellung von Toluylendiamin durch Hydrierung von Dinitrotoluol in der Gasphase umfassend die Schritte
(I) Einsprühen eines Dinitrotoluol umfassenden Stromes (**1, 11, 12**) in einen wasserstoffhaltigen Trägergasstrom (**2, 21, 22**) in einer Verdampfungsapparatur (**1000**, **1010, 1020)** wobei
a) die Temperatur des Dinitrotoluol umfassenden Stromes (**1, 11, 12**) 70 °C bis 150 °C und die Temperatur des wasserstoffhaltigen Trägergasstroms (**2**, **21, 22**) 140 °C bis 300 °C beträgt,
b) der absolute Druck des Dinitrotoluol umfassenden Stromes (**1, 11, 12**) 3,0 bar bis 30 bar und der absolute Druck des wasserstoffhaltigen Trägergasstroms (**2, 21, 22**) 1,0 bar bis 10 bar beträgt, wobei der Druck des Dinitrotoluol umfassenden Stromes (**1, 11, 12**) höher ist als der des wasserstoffhaltigen Trägergasstroms (**2, 21, 22**),
c) das molare Verhältnis von Wasserstoff zu DNT 6,0 : 1 bis 900 : 1 beträgt, sodass ein Dinitrotoluol und Wasserstoff umfassender Strom (**3, 31, 32**) erhalten wird;
(II) Abtrennung oder gezielte Zersetzung der in dem Dinitrotoluol und Wasserstoff umfassenden Strom aus Schritt (I) (**3, 31, 32**) enthaltenen Flüssigkeitsstropfen unter Erhalt eines an Flüssigkeitstropfen abgereicherten Dinitrotoluol und Wasserstoff umfassenden Gasstroms (**4, 41, 42**);
(III) Umsetzung des in dem an Flüssigkeitstropfen abgereicherten Dinitrotoluol und Wasserstoff umfassenden Gasstroms (**4, 41, 42**) enthaltenen Dinitrotoluols mit Wasserstoff in wenigstens einem Reaktionsraum (**3000, 3010, 3020**) in Gegenwart eines Katalysators (**100, 110, 120**) bei einem absoluten Druck von 1,0 bar bis 10 bar, einer Temperatur von 140 °C bis 300 °C und einer Verweilzeit im Reaktionsraum von 0,1 s bis 10 s, sodass ein Toluylendiamin enthaltender Gasstrom (**5, 51, 52**) erhalten wird,
(IV) Trennung des in Schritt (III) nach Durchlaufen des letzten Reaktionsraums (**3000, 3020**) erhaltenen Toluylendiamin enthaltenden Gasstroms (**5, 52**) in eine Toluylendiamin umfassende Flüssigphase (**6**) und eine Wasserstoff umfassende Gasphase (**7**) durch Kondensation,
(V) Rückführung wenigstens eines Teils der in Schritt (IV) erhaltenen Wasserstoff umfassenden Gasphase (**7**) in die erste Verdampfungsapparatur (**1000, 1010**) von Schritt (I).

2. Verfahren nach Anspruch 1, bei dem das Einsprühen des Dinitrotoluol umfassenden Stromes (**1, 11, 12**) in den wasserstoffhaltigen Trägergasstrom (**2, 21, 22**) in Schritt (I) mittels wenigstens einer Zweistoffdüse (**7000**) erfolgt, durch die zusätzlich zum Dinitrotoluol umfassenden Strom (**1, 11, 12**) ein Strom eines Zerstäubungsgases **9** geleitet wird, das gegenüber dem in der Umgebung auf der Düsenaustrittsseite herrschenden absoluten Druck unter einem 1,0 bar bis 20 bar höheren Druck steht.

3. Verfahren nach Anspruch 2, bei dem als Zerstäubungsgas **9** Wasserdampf, Stickstoff, Frischwasserstoff **200,** ein Teil der Wasserstoff umfassenden Gasphase **7,** ein Teil des wasserstoffhaltigen Trägergasstromes (**2, 21, 22**) oder eine Mischung aus zwei oder mehr der vorgenannten Gase eingesetzt wird.

4. Verfahren nach einem der Ansprüche 1 bis 3, bei dem die Kondensation in Schritt (IV) fraktionierend mit sukkzesive sinkender Kondensationstemperatur durchgeführt wird, sodass mehrere Toluylendiamin umfassende Flüssigphasen (**6a, 6b, 6c**) erhalten werden.

5. Verfahren nach einem der Ansprüche 1 bis 4, bei dem
(VI) die in Schritt (IV) erhaltene Toluylendiamin umfassende Flüssigphase **6** oder die Toluylendiamin umfassenden Flüssigphasen (**6a, 6b, 6c**) destillativ aufgearbeitet wird/werden, um Rein-Toluylendiamin zu gewinnen.

6. Verfahren nach Anspruch 5, bei dem die Kondensation in Schritt (IV) fraktionierend mit sukzessive sinkender Kondensationstemperatur durchgeführt wird, sodass mehrere Toluylendiamin umfassende Flüssigphasen (**6a, 6b, 6c**) erhalten werden, die in Schritt (VI) getrennt voneinander in verschiedene Stellen der destillativen Aufarbeitung geleitet werden.

7. Verfahren nach einem der Ansprüche 1 bis 6, bei dem das Dinitrotoluol im Dinitrotoluol umfassenden Strom (**1**, **11**, **12**) die folgende Zusammensetzung aufweist:
99,00 Massen-% bis 99,94 Massen-% Dinitrotoluol,
> 5 ppm Nitrokresole,
> 20 ppm Trinitrotoluol,
> 500 ppm sonstige Verunreinigungen umfassend Wasser, Mononitrotoluol, Schwefelsäure und Salze der Schwefelsäure,
jeweils bezogen auf die Gesamtmasse des Dinitrotoluols im Dinitrotoluol umfassenden Strom (**1, 11**, **12**).

## Claims

1. A continuous process for preparing toluenediamine by hydrogenation of dinitrotoluene in the gas phase, which comprises the steps
(I) spraying of a dinitrotoluene-comprising stream (**1, 11, 12**) into a hydrogen-containing carrier gas stream (**2, 21, 22**) in a vaporization apparatus (**1000, 1010, 1020**), where
a) the temperature of the dinitrotoluene-comprising stream (**1, 11, 12**) is from 70°C to 150°C and the temperature of the hydrogen-containing carrier gas stream (**2, 21, 22**) is from 140°C to 300°C,
b) the absolute pressure of the dinitrotoluene-comprising stream (**1, 11, 12**) is from 3.0 bar to 30 bar and the absolute pressure of the hydrogen-containing carrier gas stream (**2, 21, 22**) is from 1.0 bar to 10 bar, where the pressure of the dinitrotoluene-comprising stream (**1, 11, 12**) is higher than that of the hydrogen-containing carrier gas stream (**2, 21, 22**),
c) the molar ratio of hydrogen to DNT is from 6.0:1 to 900:1,
so as to give a stream (**3, 31, 32**) comprising dinitrotoluene and hydrogen;
(II) removal or targeted decomposition of the liquid droplets present in the stream (**3, 31, 32**) comprising dinitrotoluene and hydrogen from step (I) to give a gas stream (**4, 41, 42**) which comprises dinitrotoluene and hydrogen and has been depleted in liquid droplets;
(III)reaction of the dinitrotoluene present in the gas stream (**4, 41, 42**) which comprises dinitrotoluene and hydrogen and has been depleted in liquid droplets with hydrogen in at least one reaction space (**3000, 3010**, **3020**) in the presence of a catalyst (**100, 110, 120**) at an absolute pressure of from 1.0 bar to 10 bar, a temperature of from 140°C to 300°C and a residence time in the reaction space of from 0.1 s to 10 s, so as to give a toluenediamine-containing gas stream (**5, 51, 52**),
(IV) separation of the toluenediamine-containing gas stream (**5, 52**) obtained in step (III) after passing through the last reaction space (**3000, 3020**) into a toluenediamine-comprising liquid phase (**6**) and a hydrogen-comprising gas phase (**7**) by condensation;
(V) recirculation of at least part of the hydrogen-comprising gas phase (**7**) obtained in step (IV) into the first vaporization apparatus (**1000, 1010**) of step (I).

2. The process as claimed in claim 1, wherein the spraying of the dinitrotoluene-comprising stream (**1, 11, 12**) into the hydrogen-containing carrier gas stream (**2, 21, 22**) in step (I) is carried out by means of at least one two-fluid nozzle (**7000**) through which a stream of an atomizing gas **9** which is under a pressure which is from 1.0 bar to 20 bar higher than the absolute pressure prevailing in the surroundings on the nozzle exit side is passed in addition to the dinitrotoluene-comprising stream (**1, 11, 12**).

3. The process as claimed in claim 2, wherein steam, nitrogen, fresh hydrogen **200,** part of the hydrogen-comprising gas phase **7,** part of the hydrogen-containing carrier gas stream (**2, 21, 22**) or a mixture of two or more of the abovementioned gases is used as atomizing gas **9.**

4. The process as claimed in any of claims 1 to 3, wherein the condensation in step (IV) is carried out fractionally with a gradually decreasing condensation temperature so that a plurality of toluenediamine-comprising liquid phases (**6a, 6b, 6c**) are obtained.

5. The process as claimed in any of claims 1 to 4, wherein
(VI) the toluenediamine-comprising liquid phase **6** or the toluenediamine-comprising liquid phases (**6a, 6b, 6c**) obtained in step (IV) is/are worked up by distillation in order to obtain pure toluenediamine.

6. The process as claimed in claim 5, wherein the condensation in step (IV) is carried out fractionally with a gradually decreasing condensation temperature so as to give a plurality of toluenediamine-comprising liquid phases (**6a, 6b, 6c**) which are, in step (VI), introduced separately from one another into various places in the work-up by distillation.

7. The process as claimed in any of claims 1 to 6, wherein the dinitrotoluene in the dinitrotoluene-comprising stream (**1, 11, 12**) has the following composition:
from 99.00% by mass to 99.94% by mass of dinitrotoluene,
> 5 ppm of nitrocresols,
> 20 ppm of trinitrotoluene,
> 500 ppm of other impurities comprising water, mononitrotoluene, sulfuric acid and salts of sulfuric acid,
in each case based on the total mass of the dinitrotoluene in the dinitrotoluene-comprising stream (**1, 11, 12**).

## Revendications

1. Procédé continu de fabrication de toluylène-diamine par hydrogénation de dinitrotoluène dans la phase gazeuse, comprenant les étapes suivantes :
(I) l'injection d'un courant comprenant du dinitrotoluène (1, 11, 12) dans un courant de gaz vecteur contenant de l'hydrogène (2, 21, 22) dans un appareil d'évaporation (1000, 1010, 1020),
a) la température du courant comprenant du dinitrotoluène (1, 11, 12) étant de 70 °C à 150 °C et la température du courant de gaz vecteur contenant de l'hydrogène (2, 21, 22) étant de 140 °C à 300 °C,
b) la pression absolue du courant comprenant du dinitrotoluène (1, 11, 12) étant de 3,0 bar à 30 bar et la pression absolue du courant de gaz vecteur contenant de l'hydrogène (2, 21, 22) étant de 1,0 bar à 10 bar, la pression du courant comprenant du dinitrotoluène (1, 11, 12) étant supérieure à celle du courant de gaz vecteur contenant de l'hydrogène (2, 21, 22),
c) le rapport molaire entre l'hydrogène et le DNT étant de 6,0:1 à 900:1,
de manière à obtenir un courant comprenant du dinitrotoluène et de l'hydrogène (3, 31, 32) ;
(II) la séparation ou la décomposition ciblée des gouttes de liquide contenues dans le courant comprenant du dinitrotoluène et de l'hydrogène (3, 31, 32) de l'étape (I) pour obtenir un courant gazeux comprenant du dinitrotoluène et de l'hydrogène appauvri en gouttes de liquide (4, 41, 42) ;
(III) la mise en réaction du dinitrotoluène contenu dans le courant gazeux comprenant du dinitrotoluène et de l'hydrogène appauvri en gouttes de liquide (4, 41, 42) avec de l'hydrogène dans au moins une chambre de réaction (3000, 3010, 3020) en présence d'un catalyseur (100, 110, 120) à une pression absolue de 1,0 bar à 10 bar, à une température de 140 °C à 300 °C et pendant un temps de séjour dans la chambre de réaction de 0,1 s à 10 s, de manière à obtenir un courant gazeux contenant la toluylène-diamine (5, 51, 52),
(IV) la séparation du courant gazeux contenant la toluylène-diamine (5, 52) obtenu à l'étape (III) après le passage dans la dernière chambre de réaction (3000, 3020) en une phase liquide contenant de la toluylène-diamine (6) et une phase gazeuse comprenant de l'hydrogène (7) par condensation,
(V) le recyclage d'au moins une partie de la phase gazeuse comprenant de l'hydrogène (7) obtenue à l'étape (IV) dans le premier appareil d'évaporation (1000, 1010) de l'étape (I).

2. Procédé selon la revendication 1, dans lequel l'injection du courant comprenant du dinitrotoluène (1, 11, 12) dans le courant de gaz vecteur contenant de l'hydrogène (2, 21, 22) à l'étape (I) a lieu au moyen d'au moins une buse à deux composants (7000), par laquelle en plus du courant comprenant du dinitrotoluène (1, 11, 12) un courant d'un gaz d'atomisation 9 est introduit, qui se trouve à une pression supérieure de 1,0 bar à 20 bar par rapport à la pression absolue régnant dans l'environnement du côté de la sortie de la buse.

3. Procédé selon la revendication 2, dans lequel de la vapeur d'eau, de l'azote, de l'hydrogène frais 200, une partie de la phase gazeuse comprenant de l'hydrogène 7, une partie du courant de gaz vecteur contenant de l'hydrogène (2, 21, 22) ou un mélange de deux ou plus des gaz susmentionnés est utilisé en tant que gaz d'atomisation 9.

4. Procédé selon l'une quelconque des revendications 1 à 3, dans lequel la condensation à l'étape (IV) est réalisée sous forme fractionnée avec une température de condensation qui diminue successivement, de manière à obtenir plusieurs phases liquides comprenant de la toluylène-diamine (6a, 6b, 6c).

5. Procédé selon l'une quelconque des revendications 1 à 4, dans lequel
(VI) la phase liquide comprenant de la toluylène-diamine 6 ou les phases liquides comprenant de la toluylène-diamine (6a, 6b, 6c) obtenues à l'étape (IV) sont traitées par distillation pour obtenir de la toluylène-diamine pure.

6. Procédé selon la revendication 5, dans lequel la condensation à l'étape (IV) est réalisée sous forme fractionnée avec une température de condensation qui diminue successivement, de manière à obtenir plusieurs phases liquides comprenant de la toluylène-diamine (6a, 6b, 6c), qui sont introduites séparément les unes des autres à l'étape (VI) à différents emplacements du traitement de distillation.

7. Procédé selon l'une quelconque des revendications 1 à 6, dans lequel le dinitrotoluène dans le courant comprenant du dinitrotoluène (1, 11, 12) présente la composition suivante :
99,00 % en masse à 99,94 % en masse de dinitrotoluène,
> 5 ppm de nitrocrésols,
> 20 ppm de trinitrotoluène,
> 500 ppm d'autres impuretés comprenant de l'eau, du mononitrotoluène, de l'acide sulfurique et des sels de l'acide sulfurique,
à chaque fois par rapport à la masse totale du dinitrotoluène dans le courant comprenant du dinitrotoluène (1, 11, 12).
